(19) Europäisches Patentamt
European Patent Office
Office européen des brevets

(11) Veröffentlichungsnummer : **0 447 889 B1**

(12) **EUROPÄISCHE PATENTSCHRIFT**

(45) Veröffentlichungstag der Patentschrift :
**04.05.94 Patentblatt 94/18**

(51) Int. Cl.$^5$ : **C07C 13/48,** C07C 2/00

(21) Anmeldenummer : **91103536.8**

(22) Anmeldetag : **08.03.91**

# (54) Verfahren zur Herstellung von 1,1,3,4,4,6-Hexamethyl-1,2,3,4-tetrahydronaphthalin.

(30) Priorität : **17.03.90 DE 4008694**

(43) Veröffentlichungstag der Anmeldung :
**25.09.91 Patentblatt 91/39**

(45) Bekanntmachung des Hinweises auf die Patenterteilung :
**04.05.94 Patentblatt 94/18**

(84) Benannte Vertragsstaaten :
**CH DE ES FR GB LI NL**

(56) Entgegenhaltungen :
**US-A- 2 551 573**
**US-A- 3 856 875**
**US-A- 4 877 915**

(56) Entgegenhaltungen :
**PATENT ABSTRACTS OF JAPAN, unexamined applications, C field, Band 5, Nr. 102, 2. Juli 1981, THE PATENT OFFICE JAPANESE GOVERNMENT, Seite 19 C 61**
**PATENTS ABSTRACTS OF JAPAN, unexamined applications, C field, Band 6, Nr. 110, 22. Juni 1982, THE PATENT OFFICE JAPANESE GOVERNMENT, Seite 131 C 109**

(73) Patentinhaber : **BASF Aktiengesellschaft**
**Carl-Bosch-Strasse 38**
**D-67063 Ludwigshafen (DE)**

(72) Erfinder : **Huellmann, Michael, Dr.**
**Siegfriedstrasse 41**
**W-6148 Heppenheim (DE)**
Erfinder : **Mayr, Herbert, Prof. Dr.**
**Sandsteinstrasse 7**
**W-2401 Gross Groenau (DE)**
Erfinder : **Becker, Rainer, Dr.**
**Im Haseneck 22**
**W-6702 Bad Duerkheim (DE)**

EP 0 447 889 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein neues und verbessertes Verfahren zur Herstellung von 1,1,3,4,4,6-Hexamethyl-1,2,3,4-tetrahydronaphthalin (1,1,3,4,4,6-Hexamethyltetralin) durch Umsetzung von p-Cymol mit bestimmten Hexenisomeren in Gegenwart katalytisch wirksamer Mengen eines Aluminiumhalogenids und katalytisch wirksamer Mengen einer Triphenylmethylverbindung.

Durch Acetylierung von 1,1,3,4,4,6-Hexamethyl-1,2,3,4-tetrahydronaphthalin erhält man ein äußerst wertvolles Moschusparfüm der Tetralinreihe, das 7-Acetyl-1,1,3,4,4,6-hexamethyl-1,2,3,4-tetrahydronaphthalin (Tonalid®). 7-Acetyl-1,1,3,4,4,6-hexamethyl-1,2,3,4-tetrahydronaphthalin kommt insbesondere wegen seiner hohen Qualität und lang anhaftenden Duftnote als künstliches Moschusparfüm eine große Bedeutung zu. Darüberhinaus kommt 7-Acetyl-1,1,3,4,4,6-hexamethyl-1,2,3,4-tetrahydronaphthalin in aufgrund seiner süßlichen Geruchsnote als Ersatzstoff für die aus toxikologischen Gründen vermutlich zu ersetzenden Nitromoschusverbindungen in Betracht.

Aus der US-A-2,759,022 ist die Umsetzung von p-Cymol mit Methyl-tert.-butylcarbinol in Gegenwart eines Schwefelsäurekatalysators bekannt. Das erhaltene Reaktionsprodukt wird weder hinsichtlich seiner Strukturformel näher charakterisiert, noch werden Angaben über die Ausbeute gemacht.

Aus der US-A-2,851,501, US-A-3,246,044, US-A-3,278,621, US-A-3,379,785 und der NL-A-66 12 053 können 1,1,3,4,4,6-Hexamethyl-1,2,3,4-tetrahydronaphthalin als auch 7-Acetyl-1,1,3,4,4,6-hexamethyl-1,2,3,4-tetrahydronaphthalin über das α,p-Dimethylstyrol V

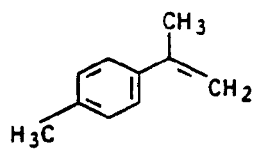

(V)

hergestellt werden. Der Nachteil dieses Verfahrens besteht darin, daß das notwendige α,p-Dimethylstyrol aus p-Cymol hergestellt werden muß.

Aus der US-A-3,856,875 und der DE-A-29 10 493 ist bekannt, 1,1,3,4,4,6-Hexamethyl-1,2,3,4-tetrahydronaphthalin durch Umsetzen von p-Cymol und Neohexen oder 2,3-Dimethyl-but-1-en und eines tert.-Alkylhalogenids in Gegenwart eines Aluminiumhalogenidkatalysators zu erhalten. Nachteilig an diesen Verfahren ist insbesondere der molare Einsatz halogenhaltiger Verbindungen wie tert.-Butylchlorid oder Ethylendichlorid. Darüberhinaus entstehen während der Cycloalkylierung molare Mengen gasförmigen Chlorwasserstoffes, die besondere Vorsichtsmaßnahmen erforderlich machen.

Aus der US-A-4,551,573 ist ein Verfahren bekannt, bei dem man, ausgehend von p-Cymol und Neohexen, befriedigende Ausbeuten an 1,1,3,4,4,6-Hexamethyl-1,2,3,4-tetrahydronaphthalin erhält, wenn man in Gegenwart katalytischer Mengen Jod arbeitet. Allerdings ist auch dieses Verfahren für eine technische Anwendung ungeeignet, da die Verwendung von Jod sehr problematisch ist (Jod-Toxizität, Korrosion von Anlagen, unerwünschte Produktfärbungen und umständliche Aufarbeitung).

Der vorliegenden Erfindung lag daher die Aufgabe zugrunde, den zuvor genannten Nachteilen abzuhelfen.

Demgemäß wurde ein neues und verbessertes Verfahren zur Herstellung von 1,1,3,4,4,6-Hexamethyl-1,2,3,4-tetrahydronaphthalin der Formel I

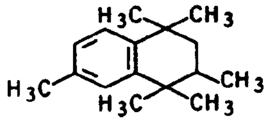

(I)

gefunden, welches dadurch gekennzeichnet ist, daß man p-Cymol der Formel II

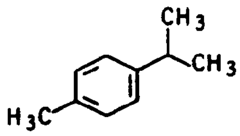

(II)

mit einem Hexen der Formel IIIa, IIIb und/oder IIIc

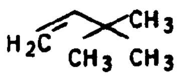 ,
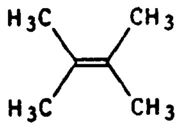 ,
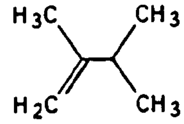

(IIIa) (IIIb) (IIIc)

in Gegenwart einer katalytischen Menge Aluminiumhalogenid und einer katalytischen Menge einer Triphenylmethylverbindung der allgemeinen Formel IV

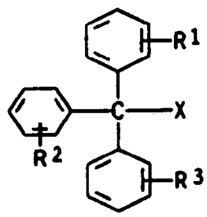

(IV),

in der $R^1$, $R^2$, $R^3$ unabhängig voneinander für Wasserstoff, $C_1$-$C_4$-Alkyl, Nitro oder Halogen und X für Wasserstoff oder Halogen, stehen, umsetzt.

Das Verfahren zur Herstellung von 1,1,3,4,4,6-Hexamethy 1-1,2,3,4-tetrahydronaphthalin I kann wie folgt durchgeführt werden:

p-Cymol (II) und die beiden Katalysatoren Aluminiumhalogenid und Triphenylmethylverbindung (IV) werden vorgelegt und das Hexen (IIIa, IIIb, IIIc) zugegeben.

p-Cymol (II) und das Hexen (IIIa, IIIb, IIIc) werden im Molverhältnis von 0,5:1 bis 5:1, vorzugsweise von 0,8:1 bis 4:1, besonders bevorzugt von 1:1 bis 3:1 umgesetzt.

Die Umsetzung wird bei einer Temperatur zwischen (-10) und 50°C, vorzugsweise zwischen 10 und 40°C, besonders bevorgzugt zwischen 15 und 30°C, durchgeführt. Die Reaktion kann in Gegenwart eines Lösungsmittels wie $C_5$-$C_{20}$-Alkan, z.B. Pentan, Hexan, Cyclohexan oder in Abwesenheit eines Lösungsmittels, bevorzugt lösungsmittel frei durchgeführt werden.

Als Aluminiumhalogenide eignen sich beispielsweise Aluminiumtrichlorid, Aluminiumtribromid und Aluminiumtrijodid. Bevorzugt verwendet man wasserfreies Aluminiumtrichlorid, Aluminiumtribromid und Aluminiumtrijodid, besonders bevorzugt wasserfreies Aluminiumtrichlorid.

Als Triphenylmethylverbindungen IV eignen sich beispielsweise Triphenylmethylchlorid, 3,3',3"-Trichlortriphenylmethylchlorid, 4,4',4"-Trichlortriphenylmethylchlorid, bevorzugt 3,3',3"-Trichlortriphenylmethylchlorid, besonders bevorzugt Triphenylmethylchlorid. Die Herstellung z.B. von Triphenylmethylchlorid findet sich beispielsweise im Beilstein, Bd. 5, S. 700 oder im Organikum, VEB Verlag 1977, S. 399.

Im allgemeinen werden Aluminiumhalogenid und p-Cymol im Molverhältnis von 0,001:1 bis 0,5:1, vorzugsweise 0,005:1 bis 0,2:1, besonders bevorzugt 0,05:1 bis 0,1:1 und Triphenylmethylverbindung und p-Cymol im Molverhältnis von 0,001:1 bis 0,1:1, vorzugsweise 0,005:1 bis 0,04:1, besonders bevorzugt 0,05:1 bis 0,02:1, umgesetzt.

Das nach dem Verfahren der Erfindung angefallene 1,1,3,4,4,6-Hexamethyl-1,2,3,4-tetrahydronaphthalin läßt sich in üblicher Weise (US 3,246,044, Bsp. II) zu dem als Moschusparfüm der Tetralinreihe bekannten 7-Acetyl-1,1,3,4,4,6-hexamethyl-1,2,3,4-tetrahydronaphthalin acetylieren.

Beispiele

Beispiel 1

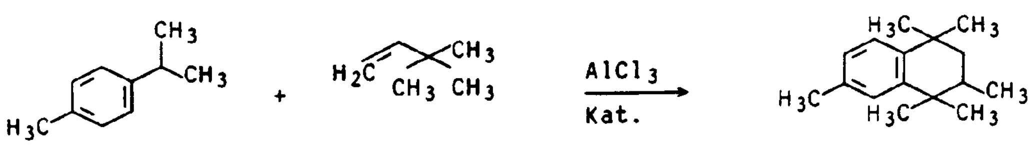


Zu 90 g (0,67 Mol) p-Cymol werden bei Raumtemperatur (20 bis 25°C) 7 g (0,052 Mol) wasserfreies Aluminiumtrichlorid und 2,78 g (0,01 Mol) Triphenylmethylchlorid hinzugefügt. Anschließend werden in 9 Minuten 85

g (1,01 Mol) Neohexen so zugetropft, daß die Reaktionstemperatur zwischen 30 und 35°C gehalten wird. Direkt nach Zulaufende wird eine Probe zur gaschromatographischen Analyse entnommen. Nach jeweils 10 Minuten werden 6 weitere Proben gaschromatographisch untersucht. Die Ergebnisse sind in Tabelle 1 zusammengestellt.

Tabelle 1 Herstellung von 1,1,3,4,4,6-Hexamethyl-1,2,3,4-tetrahydronaphthalin aus p-Cymol und Neohexen

| Reaktionszeit (min) | Umsatz (%) $AlCl_3$ + $(C_6H_5)_3CCl$ | $AlCl_3$ + $J_2$* | $AlCl_3$ | Selektivität (%) $AlCl_3$ + $(C_6H_5)_3CCl$ | $AlCl_3$ + $J_2$* | $AlCl_3$ |
|---|---|---|---|---|---|---|
| 8 | 80 | 82 | 47 | 77 | 75 | 73 |
| 18 | 81 | 82 | 56 | 76 | 75 | 73 |
| 28 | 82 | 83 | 57 | 76 | 74 | 73 |
| 38 | 83 | 83 | 64 | 76 | 74 | 72 |
| 48 | 83 | 83 | 69 | 76 | 74 | 72 |
| 58 | 83 | 83 | 72 | 74 | 74 | 72 |

*US-A-4 551 573

Beispiel 2

Zu 335 g p-Cymol (2,5 Mol) werden bei Raumtemperatur (20 bis 25°C) 17,5 g (0,13 Mol) Aluminiumtrichlorid und 8,4 g (0,03 Mol) Triphenylmethylchlorid hinzugefügt. Anschließend werden innerhalb von 45 min 420 g (5,0 Mol) Neohexen so zugetropft, daß die Reaktionstemperatur maximal ca. 35°C beträgt. Nach Zulaufende wird direkt hydrolysiert, die organische Phase abgetrennt, mit Wasser gewaschen, mit $Na_2SO_4$ getrocknet und anschließend p-Cymol-Reste und Niedersieder vom Wertprodukt abdestilliert. Im Rückstand verbleiben 520 g 1,1,3,4,4,6-Hexamethyl-1,2,3,4-tetrahydronaphthalin (61%ig). Dies entspricht einer Ausbeute von 59% bezogen auf p-Cymol.

Beispiel 3

Umsetzung von p-Cymol mit 2,3-Dimethyl-but-1-en und Triphenylmethylchlorid

Zu 40,2 g p-Cymol (0,3 Mol) werden bei Raumtemperatur 2,1 g (0,016 Mol) $AlCl_3$ und 1,0 g (3,6 mmol) Triphenylmethylchlorid hinzugefügt. Anschließend werden innerhalb von 5 Minuten 50,4 g (0,6 Mol) 2,3-Dimethylbuten-1 zugetropft (exotherme Reaktion) und direkt nach Zulaufende hydrolysiert. Nach der Aufarbeitung analog Beispiel 1 verbleiben im Rückstand 59 g (61%) 1,1,3,4,4,6-Hexamethyl-1,2,3,4-tetrahydronaphthalin.

## Patentansprüche

1. Verfahren zur Herstellung von 1,1,3,4,4,6-Hexamethyl-1,2,3,4-tetrahydronaphthalin der Formel I

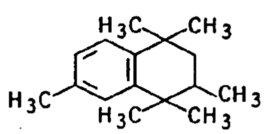

(I)

dadurch gekennzeichnet, daß man p-Cymol der Formel II

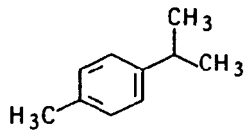

(II)

mit einem Hexen der Formel IIIa, IIIb und/oder IIIc

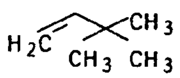

,

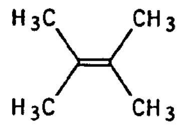

,

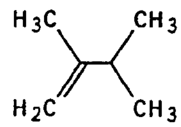

(IIIa) (IIIb) (IIIc)

in Gegenwart einer katalytischen Menge Aluminiumhalogenid und einer katalytischen Menge einer Triphenylmethylverbindung der allgemeinen Formel IV

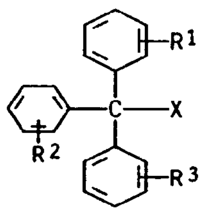

(IV),

in der $R^1$, $R^2$, $R^3$ unabhängig voneinander für Wasserstoff, $C_1$-$C_4$-Alkyl, Nitro oder Halogen und X für Wasserstoff oder Halogen, stehen, umsetzt.

2. Verfahren nach Anspruch 1, dadurch gekennzeichnet, daß man die Umsetzung bei einer Temperatur von -10°C bis 50°C durchführt.

3. Verfahren nach Anspruch 1, dadurch gekennzeichnet, daß man p-Cymol II und ein Hexen IIIa, IIIb und/oder IIIc in einem Molverhältnis von 0,5:1 bis 5:1 verwendet.

4. Verfahren nach Anspruch 1, dadurch gekennzeichnet, daß man als Aluminiumhalogenid Aluminiumtrichlorid oder Aluminiumtribromid verwendet.

5. Verfahren nach Anspruch 1, dadurch gekennzeichnet, daß man Aluminiumtrichlorid oder Aluminiumtribromid zu p-Cymol im Molverhältnis von 0,001:1 bis 0,5:1 verwendet.

6. Verfahren nach Anspruch 1, dadurch gekennzeichnet, daß man Triphenylverbindungen IV zu p-Cymol im Molverhältnis von 0,001:1 bis 0,1:1 verwendet.

## Claims

1. A process for preparing 1,1,3,4,4,6-hexamethyl-1,2,3,4-tetrahydronaphthalene of the formula I

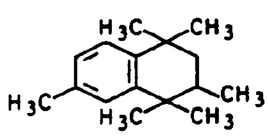

(I)

which comprises reacting p-cymene of the formula II

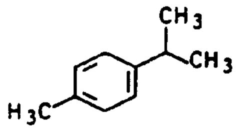

(II)

with a hexene of the formula IIIa, IIIb and/or IIIc

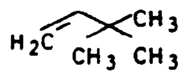 , 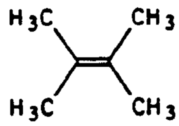 , 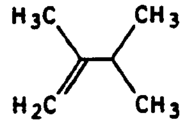

(IIIa) (IIIb) (IIIc)

in the presence of a catalytic amount of aluminum halide and a catalytic amount of a triphenylmethyl compound of the formula IV

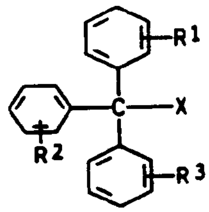

(IV),

where $R^1$, $R^2$ and $R^3$ are each, independently of one another, hydrogen, $C_1$-$C_4$-alkyl, nitro or halogen and X is hydrogen or halogen.

2. A process as claimed in claim 1, wherein the reaction is carried out at from -10°C to 50°C.

3. A process as claimed in claim 1, wherein p-cymene II and a hexene IIIa, IIIb or IIIc are used in a molar ratio of from 0.5:1 to 5:1.

4. A process as claimed in claim 1, wherein aluminum trichloride or tribromide is used as aluminum halide.

5. A process as claimed in claim 1, wherein aluminum trichloride or tribromide and p-cymene are used in the molar ratio of from 0.001:1 to 0.5:1.

6. A process as claimed in claim 1, wherein a triphenylmethyl compound IV and p-cymene are used in the molar ratio of from 0.001:1 to 0.1:1.

## Revendications

1. Procédé de préparation de 1,1,3,4,4,6-hexaméthyl-1,2,3,4-tétrahydronaphtalène de formule I

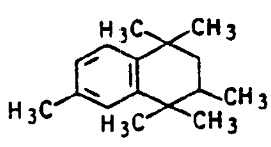

(I)

caractérisé en ce qu'on fait réagir du p-cymène de formule II

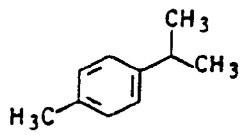

(II)

avec un hexène de formule IIIa, IIIb et/ou IIIc

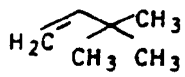

,

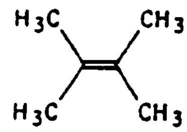

,

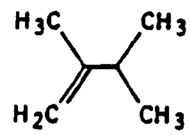

(IIIa) (IIIb) (IIIc)

en présence d'une quantité catalytique d'halogénure d'aluminium et d'une quantité catalytique d'un composé triphénylméthylique de formule générale IV

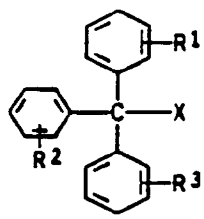

(IV),

dans laquelle $R^1$, $R^2$ et $R^3$ sont mis chacun, indépendamment les uns des autres, pour un atome d'hydrogène ou d'halogène ou pour un groupement alkyle en $C_1$-$C_4$ et X est mis pour un atome d'hydrogène ou d'halogène.

**2.** Procédé selon la revendication 1, caractérisé en ce qu'on mène la réaction à une température de -10°C à 50°C.

**3.** Procédé selon la revendication 1, caractérisé en ce qu'on utilise le p-cymène II et un hexène IIIa, IIIb et/ou IIIc dans un rapport molaire de 0,5:1 à 5:1.

**4.** Procédé selon la revendication 1, caractérisé en ce qu'on utilise, comme halogénure d'aluminium, du trichlorure d'aluminium ou du tribromure d'aluminium.

**5.** Procédé selon la revendication 1, caractérisé en ce qu'on utilise du trichlorure d'aluminium ou du tribromure d'aluminium dans un rapport molaire au p-cymène de 0,001:1 à 0,5:1.

**6.** Procédé selon la revendication 1, caractérisé en ce qu'on utilise des composés triphénylés IV dans un rapport molaire au p-cymène de 0,001:1 à 0,1:1.